# EUROPÄISCHE PATENTSCHRIFT

(11) **EP 1 228 023 B1**
(45) Veröffentlichungstag und Bekanntmachung des Hinweises auf die Patenterteilung: **28.04.2004**
(21) Anmeldenummer: 01998523.3
(22) Anmeldetag: 29.11.2001
(51) Int. Cl.: C07C 17/156, C07C 19/045

(54) **VERFAHREN ZUR HERSTELLING VON 1,2-DICHLORETHAN**
METHOD FOR PRODUCING 1,2-DICHLOROETHANE
PROCEDE DE FABRICATION DE 1,2-DICHLOROETHANE

(30) Priorität: 29.11.2000 DE 10059229; 13.02.2001 DE 10107092
(43) Veröffentlichungstag der Anmeldung: 07.08.2002
(73) Patentinhaber: Vinnolit GmbH & Co. KG, 85737 Ismaning (DE)
(72) Erfinder: GRUMANN, Helmut, 84567 Perach (DE); STÖGER, Manfred, 84508 Burgkirchen (DE); EICHLER, Jürgen, 84556 Kastl (DE); JACULI, Dieter, 84508 Burgkirchen (DE); LORK, Winfried, 50374 Erftstadt (DE); GREVE, Arend, 50374 Erftstadt (DE); WILKENS, Jan, 50354 Erfstadt (DE); KAMMERHOFER Peter, 84508 Burgkirchen (DE); TROPP Hermann, 84547 Emmerting (DE)
(74) Vertreter: Forstmeyer, Dietmar, Dr. rer. nat., Dipl.-Chem.
(86) Internationale Anmeldenummer: PCT/EP2001/013941
(87) Internationale Veröffentlichungsnummer: WO 2002/044116

(56) Entgegenhaltungen:
- DE-A- 19 703 857
- GB-A- 1 189 815

## Beschreibung

Die Erfindung bezieht sich auf ein Verfahren zur Herstellung von in Bezug auf Chloral oder/und Chloralhydrat und Kohlendioxid sehr reinem 1,2-Dichlorethan, das eine Oxichlorierung von Ethylen mit Chlorwasserstoff und einem Sauerstoff enthaltendem Gas wie Luft oder Sauerstoff sowie eine alkalische Behandlung des Dichlorethans umfasst, sowie nach diesem Verfahren hergestelltes 1,2-Dichlorethan.

Ein bekanntes Verfahren zur Herstellung von 1,2-Dichlorethan ist die Oxichlorierung von Ethylen mittels Chlorwasserstoff und Sauerstoff, wobei als unerwünschte Nebenprodukte Chloral oder/und Chloralhydrat gebildet werden. Auch enthält das Verfahrensprodukt oft größere Mengen an gelöstem Kohlendioxid, das als Nebenprodukt aus der Umsetzung von Ethylen mit Sauerstoff entsteht.

In der Druckschrift DE 1 518 931 wird ein Oxichlorierungsverfahren zur Herstellung von 1,2-Dichlorethan beschrieben, bei dem das unerwünschte Chloral mittels einer Kondensationsstufe entfernt wird. Allerdings können nur 75 bis 80 % des Chlorals durch diesen Verfahrensschritt von dem Produkt abgetrennt werden. Im weiteren wird das restliche Chloral durch Erhöhen des pH-Wertes in mittels Destillation leichter abtrennbare Substanzen, wie Chloroform, umgewandelt.

Gleichermaßen beschreibt die deutsche Patentschrift 1 468 480 ein Oxichlorierungsverfahren, bei dem mittels Alkalibehandlung die unerwünschten Nebenprodukte Chloral bzw. Chloralhydrat zu Natriumformiat und Chloroform umgewandelt werden und so leicht von dem Verfahrensprodukt 1,2-Dichlorethan abgetrennt werden können.

Der Nachteil beider Verfahren besteht darin, dass ein Großteil der alkalischen Lösung, insbesondere der wässrigen Alkalimetallhydroxid-Lösung, für die Neutralisation des Kohlendioxids verbraucht wird und eine entsprechende Menge an Alkalimetallcarbonat oder anderen Salzen entsteht. Es müssen also größere Mengen an alkalischer Lösung eingesetzt werden als zur reinen Umwandlung des Chlorals bzw. des Chloralhydrats notwendig wären. Ausserdem muss die durch die Neutralisation in erheblichem Maße auftretende Salzfracht aus der Produktionsanlage entfernt und entsorgt werden.

Die Aufgabe der vorliegenden Erfindung war es deshalb, ein Oxichlorierungsverfahren zur Herstellung von 1,2-Dichlorethan bereitzustellen, das die Nachteile der bekannten Verfahren vermeidet. Insbesondere bestand die Aufgabe der vorliegenden Erfindung darin, den Verbrauch an alkalischer Lösung sowie die Salzfracht bei der Umwandlung der unerwünschten Nebenprodukte Chloral und Chloralhydrat zu vermindern oder ganz zu vermeiden und gleichzeitig ein 1,2-Dichlorethan herzustellen, das - wenn überhaupt - nur einen geringen Gehalt an den genannten Nebenprodukten aufweist.

Diese Aufgabe wird gelöst durch ein Verfahren zur Herstellung von 1,2-Dichlorethan, das eine Oxichlorierung von Ethylen mit Chlorwasserstoff und einem Sauerstoff enthaltenden Gas wie Sauerstoff oder Luft sowie eine alkalische Behandlung des Dichlorethan enthaltenden Reaktionsgemisches umfasst, und das dadurch gekennzeichnet ist, dass Kohlendioxid insbesondere aus der 1,2-Dichlorethan enthaltenden organischen Phase entfernt wird, bevor die alkalische Behandlung des 1,2-Dichlorethans durchgeführt wird.

Es hat sich als besonders vorteilhaft herausgestellt, dass das Kohlendioxid vor der alkalischen Behandlung des 1,2-Dichlorethans möglichst weitgehend aus dem Reaktionsgemisch entfernt wird, da dadurch das Entstehen hoher Salzfrachten vermieden werden kann. Dies schont die Herstellungsanlage und spart Arbeitszeit sowie Kosten, da eine Salzfracht nicht mehr oder nur noch in vermindertem Umfang aus der Herstellungsanlage entfernt werden muss.

Vorteilhafter Weise kann durch das erfindungsgemäße Verfahren außerdem die einzusetzende Menge an alkalischen Lösungen vermindert werden. Durch den verminderten Läugenbedarf können die Herstellungskosten vermindert werden. Auch unter Gesundheitsaspekten ist dies vorteilhaft und wünschenswert, da vom Bedienpersonal weniger Volumina Lauge gehandhabt werden müssen. Weiterhin ist somit weniger Lauge zu entsorgen. Dies stellt sowohl unter Kostenaspekten als auch unter Umweltaspekten einen erheblichen Vorteil dar.

Reines bzw. sehr reines EDC weist erfindungsgemäß pro kg EDC einen Gehalt an 2-Chlorethanol von < 100 mg, insbesondere < 50 mg, vorzugsweise < 20 mg, bevorzugt < 10 mg, stärker bevorzugt < 5 mg und besonderes bevorzugt < 2 mg auf.

Sehr reines EDC weist erfindungsgemäß pro kg EDC einen Gehalt an Chloral von < 20 mg, bevorzugt < 10 mg und besonderes bevorzugt < 5 mg auf.

Sehr reines EDC weist erfindungsgemäß pro kg EDC einen Gehalt an Eisenchlorid von < 20 mg, bevorzugt < 10 mg, stärker bevorzugt < 5 mg und besonderes bevorzugt < 1 mg auf.

Vorzugsweise umfasst das erfindungsgemäße Verfahren gemäß einer Ausführungsform folgende Verfahrensschritte:
1. Oxichlorieren von Ethylen mit Chlorwasserstoff und Sauerstoff,
2. Quenchen, Waschen und/oder Abkühlen und Kondensieren der Reaktionsgase, also der aus der Oxichlorierung erhaltenen Produkte,
3. gegebenenfalls Abtrennung von der Waschflüssigkeit,
4. Destillation und Kondensation von 1,2-Dichlorethan und gegebenenfalls von Wasser aus der Quenche,
5. Trennen der wässrigen von der organischen Phase aus Stufe 4, gegebenenfalls nach Überführen des das 1,2-Dichlorethan enthaltenden Kondensats in einen Trennbehälter,
   gegebenenfalls Rezyklisieren der wässrigen Phase in die Quenche, und
   gegebenenfalls Rezyklisieren von Gasen zu der Oxichlorierungsreaktion,
6. Entfernen von Kohlendioxid aus der das 1,2-Dichlorethan enthaltenden Phase (dem Reaktionsgemisch),
7. alkalische Behandlung der das 1,2-Dichlorethan enthaltenden Phase, z.B. mit einer wässrigen Lauge,
8. Trennen der wässrigen von der organischen Phase, gegebenenfalls nach Überführen der das 1,2-Dichlorethan enthaltenden Phase aus Stufe 7 in einen Trennbehälter, gegebenenfalls Rezyklisieren der wässrigen Phase in die Quenche,
9. Gewinnen von 1,2-Dichlorethan, und
10. gegebenenfalls weitere Auf- oder Weiterverarbeitungsschritte.

Das so gewonnene 1,2-Dichlorethan ist in Bezug auf Chloral/ Chloralhydrat und Kohlendioxid sehr rein.

Die Verfahrensbedingungen einzelner an sich bekannter Schritte des erfindungsgemäßen Verfahrens, insbesondere der Oxichlorierungsschritt sowie der alkalische Behandlungsschritt, können vorzugsweise in Übereinstimmung mit den in der Druckschrift DE 1 518 931 und dem deutschen Patent 1 468 480 beschriebenen Verfahrensbedingungen durchgeführt werden.

Vorzugsweise wird für den Oxichlorierungsschritt ein Katalysator verwendet, wobei sich hierfür CuCl₂- oder FeCl₃-Katalysatoren als besonders geeignet erwiesen haben.

Vorzugsweise wird das Reaktionsgemisch vor der Entfernung des Kohlendioxids in einen als Blasensäule ausgebildeten und eine Waschflüssigkeit enthaltenden Sumpfteil einer Wasch- oder Quenchzone eingeblasen.

Nach einer weiteren Ausführungsform des Verfahrens wird die aus der Reaktionszone austretende unter Umständen feststoffhaltige Reaktionsmischung, in der neben den gasförmig und/oder flüssig vorliegenden Produkten der Oxichlorierung des Ethylens unter Umständen auch Katalysatorabrieb vorhanden ist, durch eine Waschzone geleitet; diese kann z.B. eine Kolonne und einen als Blasensäule angeordneten unteren Sumpfteil umfassen. Dabei tritt die Reaktionsmischung vorzugsweise in den unteren Teil der Blasensäule ein, wo sie innig mit der dort enthaltenen Waschflüssigkeit in Berührung kommt und gleichzeitig quantitativ vom Katalysatorabrieb befreit wird. Dieser Verfahrensschritt kann auch als Quenchen bezeichnet werden.

Die verbrauchte Waschflüssigkeit aus dem Sumpfteil der Waschzone wird abgeleitet, beispielsweise neutralisiert und einer Abwasserbehandlung zugeführt.

Der gasförmige Anteil des in der Waschzone gekühlten und gewaschenen Reaktionsgemisches wird über eine Gasleitung in eine Kondensationszone, in der vorzugsweise erhöhter Druck und niedrige Temperatur herrschen, eingeleitet.

Dort wird das EDC enthaltende Reaktionsgemisch vorzugsweise unter Druck im wesentlichen kondensiert und von den flüchtigen Nebenprodukten abgetrennt. Im Kondensat liegt das Kohlendioxid gelöst vor.

Das Kohlendioxid, welches insbesondere in der das 1,2-Dichlorethan enthaltenden organischen Phase vorliegt, kann mit jeder geeigneten Verfahrensweise bzw. Vorrichtung abgetrennt werden. Vorzugsweise erfolgt das Abtrennen des Kohlendioxids von der 1,2-Dichlorethan enthaltenden Phase zumindest auch durch Entspannen der Phase z.B. in einen Behälter (Desorptionsbehälter). Dabei weist die Phase vor Entspannen z.B. einen Druck im Bereich von etwa 4 bar abs. auf, während sie nach Entspannen z.B. einen Druck im Bereich von etwa 1,1 bar abs. aufweist. Ein derartiger Behälter weist vorzugsweise einen Auslass zur Ableitung des Kohlendioxids (gasförmig) sowie einen Auslass für die das 1,2-Dichlorethan enthaltende Phase (flüssig) auf. Über diesen Auslass kann diese flüssige Phase den weiteren Verfahrensschritten zugeführt werden kann. Ein weiterer Verfahrensschritt ist die alkalische Behandlung des, in Bezug auf CO₂, bereits sehr reinen Dichlorethans zur Entfernung von Chloral und/oder Chloralhydrat.

In einer weiteren bevorzugten Ausführungsform wird das Kohlendioxid von der 1,2-Dichlorethan enthaltenden Phase in einer Kolonne durch Einleiten eines Inertgases abgetrennt. Als Inertgas kann jedes in diesem Verfahrensschritt geeignete Inertgas verwendet werden. Vorzugsweise ist das Inertgas Stickstoff. Vorteilhafter Weise werden 1,2-Dichlorethan und Inertgas im Gegenstrom aneinander vorbeigeführt, wodurch sich der Stoffaustausch verbessern lässt. Es ist aber auch eine Verfahrensführung im Gleichstrom möglich.

In noch einer weiteren bevorzugten Ausführung wird das 1,2-Dichlorethan bzw. die 1,2-Dichlorethan enthaltenden Phasen vor der Abtrennung des Kohlendioxides durch Wärmeeintrag aufgeheizt, wobei die üblichen dem Fachmann bekannten Verfahrensweisen und Vorrichtungen verwendet werden können. Besonders bevorzugt wird dieser Verfahrensschritt mittels eines Wärmetauschers durchgeführt.

Die vorstehend erwähnten Verfahren zur Kohlendioxidentfernung können auch in beliebiger Weise miteinander kombiniert werden.

Durch die erfindungsgemäße Kohlendioxid-Abtrennung wird es möglich, aus dem das 1,2-Dichlorethan enthaltenden Reaktionsgemisch, insbesondere der organischen Phase, das darin gelöste Kohlendioxid vollständig bzw. im wesentlichen vollständig zu entfernen. Vorzugsweise weist die 1,2-Dichlorethan enthaltende organische Phase nach der Kohlendioxid-Abtrennung einen Kohlendioxid-Gehalt von weniger als 0,3 % (m/m), vorzugsweise von weniger als 0,2 % (m/m) und besonders bevorzugt von weniger als 0,06 % (m/m) auf.

Mit dem erfindungsgemäßen Verfahren wird mittels einfacher technischer Mittel und auf kostengünstige Weise eine wirkungsvolle Entfernung des Katalysatorabriebs und/oder des Kohlendioxids aus der das 1,2-Dichlorethan enthaltenden Phase möglich. Erst das erfindungsgemäße Verfahren führt in vorteilhafter Weise zu einer signifikanten Kohlendioxid-Abtrennung, die einen verminderten Baseneinsatz bei wirksamem Chloral- bzw. Chloralhydrat-Abbau ermöglicht und damit eine verminderte Salzfracht zur Folge hat.

In einer besonders bevorzugten Ausführungsform wird das Produkt aus der Oxichlorierung, das das 1,2-Dichlorethan enthält, vor der Kohlendioxid-Abtrennung gequencht.

Quenchen im Sinn der Erfindung, z.B. in Form einer Abkühlung und Kondensation, heißt, dass nicht umgesetzte Edukte, wie z.B. Chlorwasserstoff, mittels geeigneter Flüssigkeiten, Lösungen, Gase oder Gasgemische vollständig oder zumindest im wesentlichen entfernt werden. Ein erfindungsgemäßes Quenchen hat den Vorteil, dass im Oxichlorierungsschritt alle oder die überwiegende Menge nicht umgesetzter Edukte aus der Phase entfernt werden, die den weiteren Verfahrensschritten unterzogen wird.

Vorzugsweise enthält das Verfahrensprodukt aus der Oxichlorierung nach dem Quenchen weniger als 0,010 % (m/m) Chlorwasserstoff, vorzugsweise weniger als 0,005 % (m/m)und am meisten bevorzugt weniger als 0,001 % (m/m). Somit können in vorteilhafter Weise Störreaktionen (Korrosion) vermindert oder sogar ganz vermieden werden.

Besonders bevorzugt erfolgt das Quenchen durch Inkontaktbringen mit einer wässrigen Lösung (Waschlösung), am meisten bevorzugt mit Wasser oder einer wässrigen Alkalilösung.

Die nach der Kohlendioxid-Abtrennung durchzuführende alkalische Behandlung der das 1,2-Dichlorethan enthaltenden Phase kann mit jeder geeigneten Verfahrensweise und mittels üblicher Vorrichtungen erfolgen. Vorzugsweise wird dieser Verfahrensschritt mit einer wässrigen alkalischen Lösung durchgeführt. Dabei weist die wässrige alkalische Lösung vorzugsweise einen pH-Wert von mehr als 8,5, bevorzugt von mehr als 9,5, auf.

Anschließend können weitere übliche dem Fachmann bekannte Trennverfahren, wie eine Destillation, durchgeführt werden, um die Chloral- bzw. Chloralhydrat-Abbauprodukte und weitere Bestandteile von dem 1,2-Dichlorethan zu trennen.

Die organischen und wässrigen Abfallprodukte des Verfahrens werden aus dem Verfahrenskreislauf abgeführt und in erforderlicher Weise entsorgt oder rezykliert. Vorzugsweise wird die aus dem letzten Verfahrensschritt gewonnene wässrige alkalische Lösung rezyklisiert, besonders bevorzugt mit weiteren wässrigen Phasen, die aus anderen Verfahrensschritten hervorgehen. Rezyklisieren im Sinn der Erfindung bedeutet, dass Verfahrensprodukte,-zwischenprodukte oder -hilfsstoffe wieder in einen Verfahrensschritt zurückgeführt und eingesetzt werden.

In einer besonders bevorzugten Ausführungsform wird die wässrige alkalische Lösung alleine oder zusammen mit den weiteren wässrigen Phasen in die Quenche rezyklisiert.

Durch das Rezyklisieren der alkalischen Lösung in die Quenche wird der pH-Wert der Quenche erhöht, wodurch das Auswaschen des nicht umgesetzten Chlorwasserstoffs aus der Oxichlorierung in vorteilhafter Weise verbessert werden kann. Ferner kann eine zusätzliche Neutralisation der Lauge zumindest teilweise entfallen.

Das mit dem erfindungsgemäßen Verfahren erhaltene Verfahrensprodukt 1,2-Dichlorethan ist im wesentlichen frei von Chloral und Chloralhydrat. Es weist vorzugsweise einen Chloral- oder/und Chloralhydrat-Gehalt von weniger als 0,02 % (m/m), vorzugsweise von weniger als 0,005 % (m/m) und besonders bevorzugt von weniger als 0,002 % (m/m) auf.

In einem weiteren Aspekt betrifft die Erfindung ein mit Bezug auf Chloral oder/und Chloralhydrat sehr reines 1,2-Dichlorethan, das mittels einem wie oben beschriebenen Verfahren erhältlich ist.

Ein erfindungsgemäßes 1,2-Dichlorethan kann in vorteilhafter Weise in Anwendungen eingesetzt werden, bei denen Chloral oder Chloralhydrat einen negativen Einfluss ausüben würde.

Weitere Vorteile und Weiterbildungen der Erfindung ergeben sich aus den Patentansprüchen, der Zeichnung und der nachfolgenden Beschreibung, in der unter Bezugnahme auf die Zeichnungen Ausführungsbeispiele der Erfindung im einzelnen beschrieben sind.

Es zeigt dabei:
Fig. 1 ein Flussdiagramm eines erfindungsgemäßen Verfahrens gemäß einer ersten bevorzugten Ausführungsform,
Fig. 2 ein Flußdiagramm einer weiteren bevorzugten Ausführungsform des erfindungsgemäßen Verfahrens und
Figur 3 ein Flußdiagramm einer weiteren bevorzugten Ausführungsform des Verfahrens

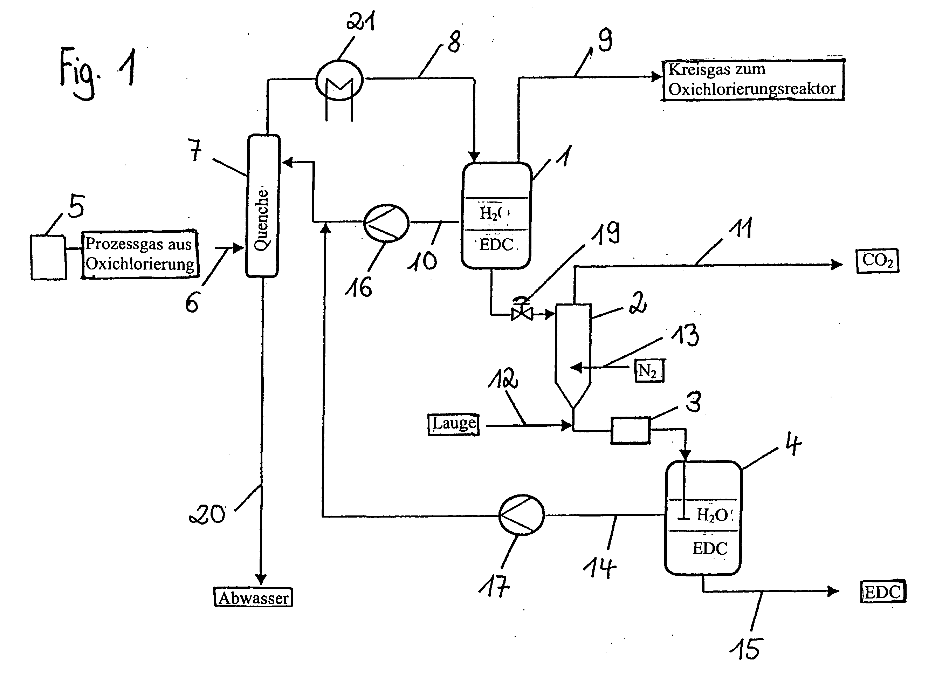

In der Fig. 1 wird ein Flussdiagramm eines erfindungsgemäßen Verfahrens dargestellt. Dabei ist mit EDC das herzustellende 1,2-Dichlorethan und mit 5 eine Oxichlorierungsstufe bezeichnet. Diese Oxichlorierung wird unter üblichen, dem Fachmann bekannten Bedingungen auf bekannte Art und Weise durchgeführt. Das Prozessgas der Oxichlorierungsstufe 5 wird über eine Leitung 6 in eine Quenchstufe 7 überführt. Das daraus erhaltene Reaktionsgemisch wird über eine Leitung 8 mit Wärmetauscher 21 in einen Dekanter 1 überführt, während das Abwasser über Abwasserleitung 20 aus der Quenchstufe 7 abgeleitet wird. Kreisgase werden aus dem Dekanter 1 über eine Leitung 9 zur Oxichlorierungsstufe 5 rezyklisiert.

Im Dekanter 1 wird die wässrige Phase abgeschieden, die über eine Leitung 10 mittels einer Pumpe 16 zur Quenchstufe 7 rezyklisiert wird. Die 1,2-Dichlorethan (EDC) enthaltende organische Phase aus dem Dekanter 1 wird in einem Behälter 2 über ein Regelventil 19 entspannt, wobei über eine Kohlendioxid-Ableitung 11 das austretende Kohlendioxid entfernt wird. Es kann dabei gemäß der gezeigten bevorzugten Ausführungsform zusätzlich Inertgas, hier Stickstoff, über eine Inertgaszuführung 13 eingespeist werden.

Danach wird in einer Vorrichtung 3 die 1,2-Dichlorethan enthaltende Phase durch Einleiten von Lauge über eine Zuleitung 12 behandelt. In einem weiteren Dekanter 4 wird die wässrige alkalische Phase von der organischen 1,2-Dichlorethan enthaltenden Phase getrennt und über eine Leitung 14 mittels einer Pumpe 17 in die Quenchstufe 7 rezyklisiert. Schließlich wird dadurch in Bezug auf Chloral oder/und Chloralhydrat sehr reines 1,2-Dichlorethan aus der Endprodukt-Ableitung 15 gewonnen.

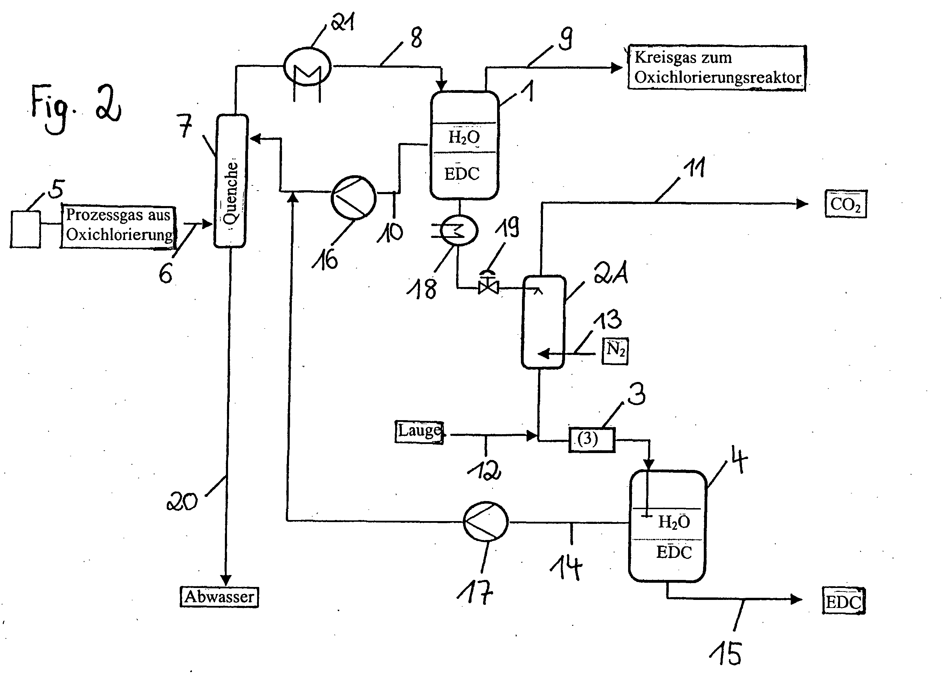

In Fig. 2 ist ein der Fig. 1 entsprechendes Flussdiagramm gemäß einer weiteren bevorzugten Ausführungsform der Erfindung dargestellt. Der Unterschied zum in der Fig. 1 dargestellten Verfahren besteht dabei darin, dass die Kohlendioxidentfernung mittels einer Kolonne 2A in Verbindung mit einem Wärmetauscher 18 unter Zuleitung von Inertgas, hier Stickstoff, im Gegenfluss erfolgt, wobei der Wärmetauscher 18 vor dem Regelventil 19 sitzt. Ansonsten entspricht der Verfahrensablauf dem in der Fig. 1 dargestellten, weswegen entsprechende Teile und Verfahrensschritte mit der Fig. 1 entsprechenden Bezugszeichen versehen wurden.

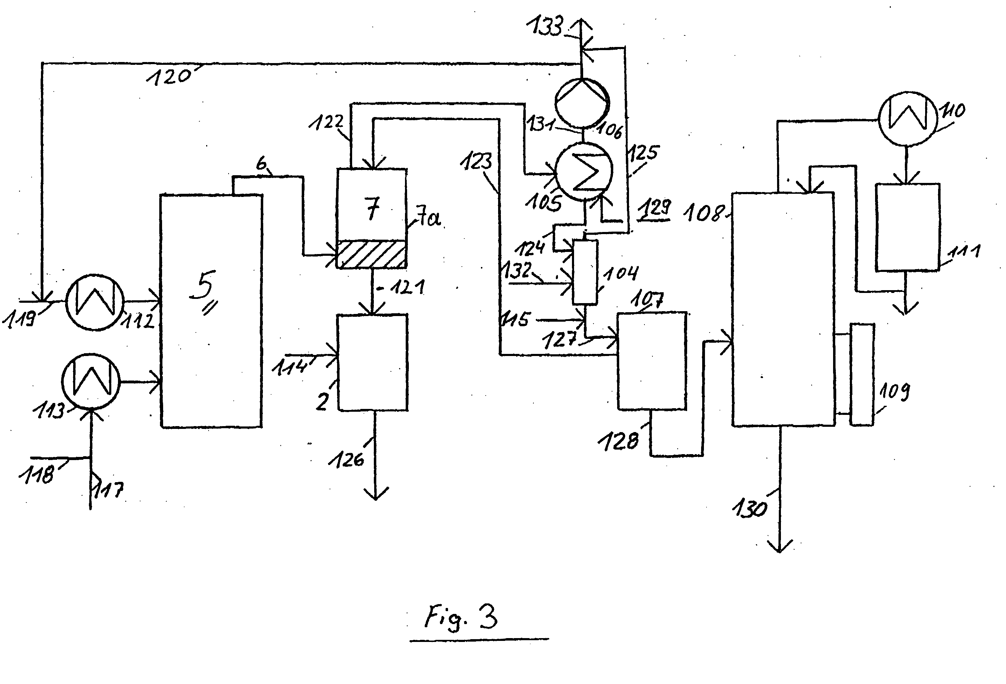

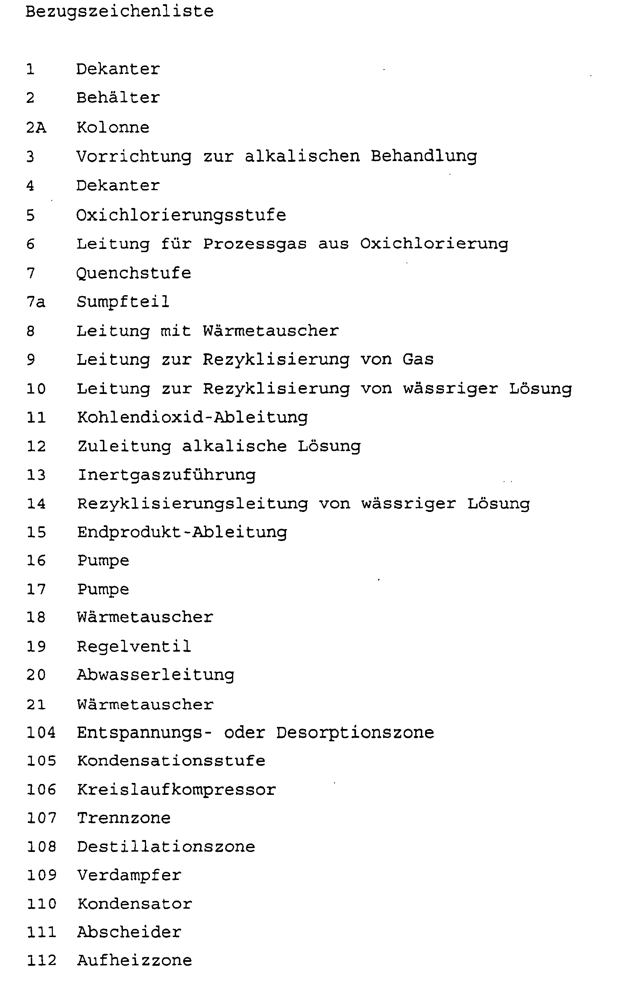

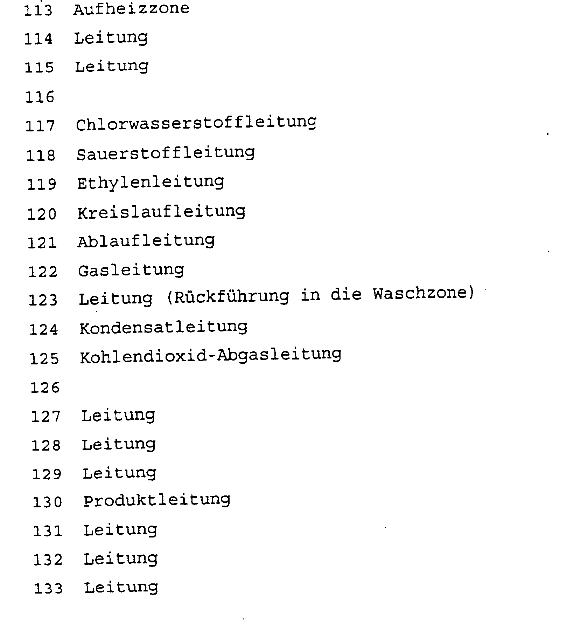

In Figur 3 ist eine Oxichlorierungsstufe 5, eine Waschoder Quenchzone 7, ein Behälter 2, eine Kondensationsstufe 105, eine Desorptionszone 104, eine Trennzone 107 und eine Destillationszone 108 als zentrale Zonen zu sehen. In zwei Aufheizzonen 112 und 113 werden die Prozessgase Chlorwasserstoff, Sauerstoff, Ethylen und Kreislaufgas, also durch Rezyklieren gewonnenes Gas vorgewärmt und der Oxichlorierungsstufe 5 zugeleitet.

Der zweiten Aufheizzone 113 werden 6000Nm³/h Chlorwasserstoff durch die Chlorwasserstoffleitung 117 und 1545 Nm³/h Sauerstoff durch die Sauerstoffleitung 118 zugeführt und in die Oxichlorierungsstufe 5 mit einer Temperatur von 140°C eingeblasen. Der ersten Aufheizzone 112 werden 10000 Nm³/h Kreislaufgas durch die Kreislaufleitung 120 und 3000Nm³/h Ethylen durch die Ethylenleitung 119 zugeführt und ebenfalls in die Oxichlorierungsstufe 5 mit einer Temperatur von 140°C eingeblasen.

Die Oxichlorierungsstufe 5 umfasst einen Wirbelschichtreaktor mit Dampferzeuger zur Abführung der Reaktionswärme; das abreagierte Reaktionsgas strömt aus dem Wirbelschichtreaktor mit einer Temperatur von 210°C durch die Reaktionsgasleitung 6 in den unteren Teil der Quenchoder Waschzone 7. Die Waschzone 7 umfasst eine Kolonne (Ø 2,2m) mit 8 Ventilböden und mit einem besonders bevozugt als Blasensäule angeordneten Sumpfteil 7a. Das Reaktionsgas tritt in den unteren Teil der Blasensäule ein, wo es innig mit der Waschflüssigkeit in Berührung kommt und gleichzeitig quantitativ vom Katalysatorabrieb befreit wird. In der Waschzone 7 wird das Reaktionsgas bis auf eine Temperatur von 95°C bis 100°C durch die Waschlösung gekühlt.

Die Waschflüssigkeit am Boden der Blasensäule läuft durch die Ablaufleitung 121 in den Behälter 2 zur Neutralisation mittels der über Leitung 114 herangeführten Natronlauge ab und wird schließlich über Leitung 126 zur weiteren Behandlung in die Abwasseranlage gegeben. Die gewaschenen und gekühlten Reaktionsgase werden durch die Gasleitung 122 aus der Waschzone 7 in die Kondensationszone 105 geleitet.

Nicht kondensiertes Kreislaufgas wird durch die Leitung 131 zum Kreislaufkompressor 106 geführt und über die Leitung 120 zur Oxichlorierungsstufe 5 rückgeführt. Ein Teil des Kreislaufgases wird über die Abgasleitung 133 ausgeschleust. Über die Kondensatleitung 124 wird das unter einem Druck von ca. 4 bar verflüssigte und gelöstes Kohlendioxid enthaltende EDC-Wasser-Gemisch mit einer Temperatur von 37°C in die Desorptionszone 104 auf einen Druck von ca. 1,6 bar entspannt, wo es sich in über Kopf gehendes Kohlendioxid und am Boden ablaufendes EDC-Wasser-Gemisch aufteilt. Gegebenenfalls kann auch vorher eine Trennung von Wasser und übrigem Reaktionsgemisch durchgeführt werden.

Der Kondensationszone 105 wird 2Nm³/h Stickstoff über die Leitung 129 zur Durchmischung des Kondensates und der Entspannungszone 104 3 Nm³/h Stickstoff über Leitung 132 zur Stabilisierung des Kohlendioxidstroms am Boden zudosiert, wobei der Stickstoffzusatz auch eine effektivere oder verbesserte Entfernung des Kohlendioxids bewirkt.

Während das aus der Desorptionszone 104 über Leitung 125 abströmende Kohlendioxid als Abgas (33 Nm³/h) aus dem Prozess ausgeschleust wird, erfolgt die Ableitung des am Boden ablaufenden EDC-/Wasser-Gemisches über Leitung 127 nach Durchmischung mit 25%-iger Natronlauge aus Leitung 115 zur Auftrennung des Zweiphasengemisches in eine alkalische Wasser- und eine EDC-Phase in die Trennzone 107.

Da sowohl die Wasser- als auch die EDC-Phase kein gelöstes Kohlendioxid mehr enthält, ist nach Natronlaugenzufuhr eine Pufferung im System durch Natriumhydrogencarbonat/Natriumkarbonatbildung ausgeschlossen und eine ausgezeichnete, konstante Regulierung des vorgegebenen pH-Wertes im Bereich von 10,5 bis 13 bevorzugt im Bereich von ungefähr 12 in der Wässerphase zum Abbau von Chloral und 2-Chlorethanol jederzeit sicher gestellt.

Die Einhaltung des pH-Wertes wird durch eine kontinuierliche Messung überwacht.

Deshalb kann die so in der Trennzone 107 erhaltene wässrige Phase als Waschflüssigkeit in die Quench- oder Waschzone 102 eingeleitet werden.

Nach einer mittleren Verweilzeit im Bereich von vorzugsweise 0,5 bis 3 Stunden, bevorzugt von ca. 1 Stunde in der Trennzone 107 wird die obere wässrige alkalische Phase (mit einem eingestellten pH-Wert über 9,5) über die Leitung 123 in die Wasch-/Quenchzone 102 rückgeführt und die untere 1,2-Dichlorethanphase über Leitung 128 in die Destillationszone geleitet. Die Destillationszone 108 umfasst eine Siebbodenkolonne (Ø 2 m) mit Verdampfer 109, Kondensator 110 und Abscheider 111.

Während der Leichtsieder- und Wasseranteil aus dem Abscheider 111 ausgeschleust wird, erfolgt der Abzug des gereinigten 1,2-Dichlorethans über Leitung 130 aus dem Sumpf der Destillationszone 108 zur Vinylchloridgewinnung in der EDC-Spaltung.

Sowohl ein gewisser Anteil der wässrigen Phase aus der Leitung 123 als auch der 1,2-Dichlorethanphase aus der Leitung 128 werden zur Leitung 127 rückgeführt, um eine zusätzliche Durchmischung zu erzielen.

Nach dem Start der Anlage lag der Gehalt an Chloral, 2-Chlorethanol und Eisenchlorid im gereinigten 1,2-Dichlorethan (EDC) mit
< 2 mg 2-Chlorethanol / kg EDC
< 5 mg Chloral / kg EDC
< 1 mg Eisenchlorid/ kg EDC
unter der Nachweisbarkeitsgrenze.

Nach einer kontinuierlichen Produktionszeit von 6 Wochen veränderten sich die als Verunreinigung analysierten Werte nicht. Die Analyse im 1,2-Dichlorethan ergab die gleichen Ergebnisse wie sie auch nach dem Start der Anlage (siehe oben) erhalten worden waren.

Bei einer Produktion von ca. 13500 t EDC betrug der Zusatz an 25%-iger Natronlauge insgesamt 327 t, entsprechend einem spezifischen Verbrauch von 24,2 kg/t EDC.

Das Verfahren zeichnet sich dadurch aus, dass ohne Kreislauffahrweise der ggf. feststoffhaltigen Suspension und unabhängig von Produktionsänderungen sowie vom pH-Wert im Ablauf der Wasch- und/oder Verweilzone erfindungsgemäß das aus der Kondensationszone mit einer Temperatur von vorzugsweise 25 bis 45 °durch Entspannung erhaltene 1,2-Dichlorethan-Wasser-Gemisch nach Abtrennung von Kohlendioxid in der Desorptionszone nur einer alkalischen Behandlung mit Natronlauge bei einem einstellbaren pH-Wert von vorzugsweise > 8,5, bevorzugt > 9,5, insbesondere im Bereich von 10,5 bis 13 und einer Verweilzeit von-beispielsweise - 0,5 bis 3 Stunden in der Trennzone unterzogen werden muss, um eine effektive Reduzierung des Chloral- und 2-Chlorethanol-Gehalte im 1,2-Dichlorethan zu erzielen und damit die gewünschte EDC-Qualität dauerhaft zun gewährleisten. Der Eisenchloridanteil liegt dabei im Produkt jederzeit unter der Nachweisbarkeitsgrenze.

Nach einer bevorzugten Ausführungsform kann ein gewisser Anteil der abgetrennten wässrigen und/oder organischen Phase in die Zuleitung zur Trennzone zurückgeführt werden, damit eine zusätzliche Durchmischung erzielt wird.

### Beispiel 1

Die Verfahrensführung erfolgt hierbei wie in der Fig. 1 dargestellt. Die Oxichlorierung wird unter üblichen Verfahrensbedingungen durchgeführt, die dem Fachmann bekannt sind und die deshalb hier nicht explizit genannt werden. Die Prozessgase aus der Oxichlorierungsstufe 5, im wesentlichen 1,2-Dichlorethan, Wasser und die unerwünschten Nebenprodukte Kohlendioxid, Chloral bzw. Chloralhydrat sowie gegebenenfalls weitere Nebenprodukte, werden in einem sich anschließenden Verfahrensschritt, der Quenchstufe 7, mit einer wässrigen Lösung gequencht. Dabei werden Spuren von in der Oxichlorierung nicht umgesetztem Chlorwasserstoff und gegebenenfalls Katalysatorreste aus dem aus der Oxichlorierung erhaltenen Produktgemisch ausgewaschen.

Anschließend wird die 1,2-Dichlorethan enthaltende Phase zusammen mit Wasser aus der Quenchstufe abdestilliert und kondensiert. Dazu wird die Quenche bei einem Druck von 2 bis 4 bar und einer Temperatur von 90 bis 110 °C in einer geeigneten Destille destilliert, kondensiert und anschließend in einen Trennbehälter, vorzugsweise einen Dekanter 1, überführt. In diesem Dekanter 1 wird die wässrige von der organischen das 1,2-Dichlorethan enthaltenden Phase getrennt. Die gasförmigen Bestandteile hieraus können in die Oxichlorierungsstufe 5 rezyklisiert werden. Die wässrige Phase wird in die Quenchstufe 7 rezyklisiert und die organische Phase wird in einem Behälter 2 entspannt, wobei das Kohlendioxid im wesentlichen aus dem 1,2-Dichlorethan entweicht und durch einen Auslass des Behälters abgeleitet wird. Der Kohlendioxid-Gehalt in der erhaltenen 1,2-Dichlorethan Phase beträgt von 0,2 % bis 0,3 % (m/m) Kohlendioxid bezogen auf die das 1,2-Dichlorethan enthaltende Phase.

In dem sich daran anschließenden Verfahrensschritt wird die 1,2-Dichlorethan enthaltende organische Phase in eine nachgeschaltete Vorrichtung 3 überführt und eine alkalische Behandlung durchgeführt. Hierzu wird eine Lauge, vorzugsweise eine NaOH-Lösung mit einer Konzentration < 10 % (m/m) in die Vorrichtung 3 eingeleitet, wodurch das Chloral bzw. das Chloralhydrat abgebaut wird.

Das Gemisch wird in einen weiteren Dekanter 4 geleitet, in dem das 1,2-Dichlorethan und die alkalische, wässrige Phase voneinander getrennt werden. Die alkalische, wässrige Phase wird in die Quenchstufe 7 rezyklisiert und das mit Bezug auf Chloral/Chloralhydrat sehr reine 1,2-Dichlorethan, das einen Chloral- oder/und Chloralhydrat-Gehalt von weniger als 0,002 % bis 0,005 % (m/m) bezogen auf die das 1,2-Dichlorethan enthaltende Phase hat, wird gewonnen.

### Beispiel 2

Die Verfahrensführung erfolgt wie in Fig. 2 dargestellt. Die Verfahrensschritte und die Verfahrensführung entsprechen dabei auch denjenigen von Fig. 1 mit dem Unterschied, dass zur Abtrennung des Kohlendioxid aus der das 1,2-Dichlorethan enthaltenden Phase diese Phase aus dem ersten Trennbehälter, einem Dekanter 1, in eine Kolonne 2A geleitet wird, wobei das Kohlendioxid durch Einleiten von Stickstoff über Inertgaszuführung 13 abgetrennt wird. Nach diesem Verfahrensschritt weist die das 1,2-Dichlorethan enthaltende Phase einen Kohlendioxid-Gehalt von 0,05 % bis 0,1 % (m/m) auf.

### Beispiel 3

Die Verfahrensschritte und die Verfahrensführung entsprechen wiederum im wesentlichen denjenigen von Fig. 1, mit dem Unterschied, dass zur Abtrennung des Kohlendioxid aus der das 1,2-Dichlorethan enthaltenden Phase diese Phase aus dem ersten Trennbehälter, einem Dekanter 1, einem indirekten Wärmeeintrag mittels eines Wärmetauschers unterzogen wird, wobei zusätzlich Stickstoff in die Kolonne 2A eingeleitet wird. Nach diesem Verfahrensschritt weist die das 1,2-Dichlorethan enthaltende Phase einen Kohlendioxid-Gehalt von < 0,05 bis 0,06 % (m/m) auf.

## Patentansprüche

1. Verfahren zur Herstellung von 1,2-Dichlorethan, wobei eine Oxichlorierung von Ethylen mit Chlorwasserstoff und einem Sauerstoff enthaltenden Gas sowie eine alkalische Behandlung des Dichlorethans durchgeführt wird,
**dadurch gekennzeichnet,**
**dass** Kohlendioxid, vor der alkalischen Behandlung von 1,2-Dichlorethan, abgetrennt wird.

2. Verfahren nach Anspruch 1, **dadurch gekennzeichnet, dass** das Abtrennen des Kohlendioxids durch Entspannen des 1,2-Dichlorethan enthaltenden Reaktionsgemisches erfolgt.

3. Verfahren nach Anspruch 1 oder 2, **dadurch gekennzeichnet, dass** das Kohlendioxid in einer Kolonne (2A) durch Einleiten eines Inertgases von dem 1,2-Dichlorethan enthaltenden Reaktionsgemisch abgetrennt wird.

4. Verfahren nach einem der vorstehenden Ansprüche, **dadurch gekennzeichnet, dass** das Inertgas Stickstoff ist.

5. Verfahren nach einem der vorstehenden Ansprüche, **dadurch gekennzeichnet, dass** das Kohlendioxid durch indirekten Wärmeeintrag von dem 1,2-Dichlorethan enthaltenden Reaktionsgemisch abgetrennt wird.

6. Verfahren nach einem der vorstehenden Ansprüche, **dadurch gekennzeichnet, dass** das Reaktionsgemisch nach der Kohlendioxid-Abtrennung einen Kohlendioxid-Gehalt von weniger als 0,3 % (m/m), vorzugsweise von weniger als 0,1 % (m/m) und besonders bevorzugt von weniger als 0,06 % (m/m) aufweist.

7. Verfahren nach einem der vorstehenden Ansprüche, **dadurch gekennzeichnet, dass** das Produkt aus der Oxichlorierung, das 1,2-Dichlorethan enthält, vor der Kohlendioxid-Abtrennung gequencht, abgekühlt und/oder verflüssigt wird.

8. Verfahren nach Anspruch 7, **dadurch gekennzeichnet, dass** das Quenchen durch Einleiten in einen als Blasensäule ausgebildeten und eine Waschflüssigkeit enthaltenden Sumpfteil einer Waschzone erfolgt.

9. Verfahren nach einem der Ansprüche 7 oder 8, **dadurch gekennzeichnet, dass** das Quenchen durch Inkontaktbringen mit einer wässrigen Lösung, vorzugsweise mit Wasser, erfolgt.

10. Verfahren nach einem der vorhergehenden Ansprüche, **dadurch gekennzeichnet, dass** das 1,2-Dichlorethan enthaltende Reaktionsgemisch nach der Kohlendioxid-Abtrennung mit einer wässrigen alkalischen Lösung behandelt wird.

11. Verfahren nach Anspruch 10, **dadurch gekennzeichnet, dass** das 1,2-Dichlorethan enthaltende Reaktionsgemisch oder das 1,2-Dichlorethan nach der Behandlung mit wässriger alkalischer Lösung von der wässrigen alkalischen Lösung getrennt wird und die wässrige alkalische Lösung vor der Behandlung vorzugsweise einen pH-Wert von größer 8,5 aufweist.

12. Verfahren nach Anspruch 11, **dadurch gekennzeichnet, dass** die wässrige alkalische Lösung rezyklisiert wird.

13. Verfahren nach Anspruch 12, **dadurch gekennzeichnet, dass** die wässrige alkalische Lösung in die Quenche rezyklisiert wird.

14. Verfahren nach einem der vorhergehenden Ansprüche, **dadurch gekennzeichnet, dass** nach der Trennung der wässrigen alkalischen von der organischen Phase ein pH-Wert im Bereich von 10,5 bis 13 eingestellt wird.

## Claims

1. Process for the preparation of 1,2-dichloroethane, wherein oxychlorination of ethylene, using hydrogen chloride and an oxygen-containing gas, and alkali treatment of the dichloroethane are carried out, **characterised in that**
carbon dioxide is separated out before alkali treatment of the 1,2-dichloroethane.

2. Process according to claim 1, **characterised in that** the carbon dioxide is separated out by relieving the 1,2-dichloroethane-containing reaction mixture of pressure.

3. Process according to claim 1 or 2, **characterised in that** the carbon dioxide is separated out from the 1,2-dichloroethane-containing reaction mixture in a column (2A) by introducing an inert gas.

4. Process according to one of the preceding claims, **characterised in that** the inert gas is nitrogen.

5. Process according to one of the preceding claims, **characterised in that** the carbon dioxide is separated out from the 1,2-dichloroethane-containing reaction mixture by indirect introduction of heat.

6. Process according to one of the preceding claims, **characterised in that** the reaction mixture, after separating out of the carbon dioxide, has a carbon dioxide content of less than 0.3 % (w/w), preferably less than 0.1 % (w/w) and especially less than 0.06 % (w/w).

7. Process according to one of the preceding claims, **characterised in that** the 1,2-dichloroethane-containing product from oxychlorination is quenched, cooled and/or liquefied before separating out of the carbon dioxide.

8. Process according to claim 7, **characterised in that** quenching is carried out by means of introduction into a sump region of a washing zone, which sump region is in the form of a bubble column and contains a washing liquid.

9. Process according to one of claims 7 or 8, **characterised in that** quenching is carried out by means of bringing into contact with an aqueous solution, especially water.

10. Process according to one of the preceding claims, **characterised in that** the 1,2-dichloroethane-containing reaction mixture is treated with an aqueous alkali solution after separating out of the carbon dioxide.

11. Process according to claim 10, **characterised in that** the 1,2-dichloroethane-containing reaction mixture or the 1,2-dichloroethane is separated from the aqueous alkali solution after treatment with the aqueous alkali solution and, before the treatment, the aqueous alkali solution preferably has a pH greater than 8.5.

12. Process according to claim 11, **characterised in that** the aqueous alkali solution is recycled.

13. Process according to claim 12, **characterised in that** the aqueous alkali solution is recycled to the quench.

14. Process according to one of the preceding claims, **characterised in that**, after separation of the aqueous alkali phase from the organic phase, a pH in the range of from 10.5 to 13 is set.

## Revendications

1. Procédé destiné à la fabrication de 1,2-dichloroéthane, dans lequel il est effectué une oxychloration d'éthylène avec du gaz hydrochlorique et un gaz contenant de l'oxygène, ainsi qu'un traitement alcalin du dichloroéthane, **caractérisé en ce que** du dioxyde de carbone est séparé du 1,2-dichloroéthane avant le traitement alcalin.

2. Procédé selon la revendication 1, **caractérisé en ce que** la séparation du dioxyde de carbone est effectuée par la détente du mélange réactionnel contenant du 1,2-dichloroéthane.

3. Procédé selon la revendication 1 ou 2, **caractérisé en ce que** le dioxyde de carbone est séparé du mélange réactionnel contenant du 1,2-dichloroéthane dans une colonne (2A) par l'introduction d'un gaz inerte.

4. Procédé selon l'une quelconque des revendications précédentes, **caractérisé en ce que** le gaz inerte est de l'azote.

5. Procédé selon l'une quelconque des revendications précédentes, **caractérisé en ce que** le dioxyde de carbone est séparé du mélange réactionnel contenant du 1,2-dichloroéthane par un apport indirect de chaleur.

6. Procédé selon l'une quelconque des revendications précédentes, **caractérisé en ce que**, après la séparation du dioxyde de carbone, la teneur en dioxyde de carbone du mélange réactionnel est inférieure à 0,3 % (m/m), de préférence inférieure à 0,1 % (m/m), et de façon particulièrement préférée inférieure à 0,06 % (m/m).

7. Procédé selon l'une quelconque des revendications précédentes, **caractérisé en ce que**, avant la séparation du dioxyde de carbone, le produit obtenu de l'oxychloration, qui contient du 1,2-dichloroéthane, est soumis à un quench, refroidi et/ou liquéfié.

8. Procédé selon la revendication 7, **caractérisé en ce que** le quench est effectué par l'introduction dans une capacité de décantation agencée sous la forme d'une colonne de bullage d'une zone de lavage contenant un liquide de lavage.

9. Procédé selon la revendication 7 ou 8, **caractérisé en ce que** le quench est effectué par la mise en contact avec une solution aqueuse, de préférence avec de l'eau.

10. Procédé selon l'une quelconque des revendications précédentes, **caractérisé en ce que**, après la séparation du dioxyde de carbone, le mélange réactionnel contenant du 1,2-dichloroéthane est traité avec une solution alcaline aqueuse.

11. Procédé selon la revendication 10, **caractérisé en ce que**, après le traitement avec de la solution alcaline aqueuse, le mélange réactionnel contenant du 1,2-dichloroéthane, ou le 1,2-dichloroéthane, est séparé de la solution alcaline aqueuse, et **en ce que**, avant le traitement, la solution alcaline aqueuse a de préférence une valeur de pH supérieure à 8,5.

12. Procédé selon la revendication 11, **caractérisé en ce que** la solution alcaline aqueuse est recyclée.

13. Procédé selon la revendication 12, **caractérisé en ce que** la solution alcaline aqueuse est recyclée dans le quench.

14. Procédé selon l'une quelconque des revendications précédentes, **caractérisé en ce qu'**une valeur de pH comprise dans la plage de 10,5 à 13 est réglée après la séparation de la phase alcaline aqueuse de la phase organique.
